# EUROPEAN PATENT APPLICATION

(11) **EP 4 790 418 A1**
(43) Date of publication of application: **12.08.2026**
(21) Application number: 25382114.4
(22) Date of filing: 11.02.2025
(51) Int. Cl.: G01N 33/574, A61P 9/00, A61P 37/00, C07K 16/28, G01N 33/74, G01N 33/68

(54) **DIAGNOSIS, PROGNOSIS, MONITORING AND/OR TREATMENT OF COMORBIDITIES IN PEOPLE LIVING WITH HUMAN IMMUNODEFICIENCY VIRUS**

(71) Applicant: Servizo Galego de Saúde (SERGAS), 15703 Santiago de Compostela, A Coruña (ES)
(72) Inventor: POVEDA LÓPEZ, Eva, 15703 Santiago de Compostela (ES); LÓPEZ LÓPEZ, Aida, 15703 Santiago de Compostela (ES)
(74) Representative: Hoffmann Eitle

(57) **Abstract**

The present invention refers to the diagnosis, prognosis, monitoring and/or treatment of comorbidities in people living with human immunodeficiency virus (HIV) (PLWH).

## Description

### FIELD OF THE INVENTION

The present invention refers to the medical field. Particularly, the present invention refers to the diagnosis, prognosis, monitoring and/or treatment of comorbidities in people living with human immunodeficiency virus (HIV) (PLWH).

### STATE OF THE ART

Advances in antiretroviral therapy (ART) have significantly improved the life expectancy of people living with HIV (PLWH), bringing it closer to that of the general population. However, despite achieving virological suppression and immune restoration through ART, PLWH experience persistent immune activation and chronic systemic inflammation. This condition has been associated with a higher prevalence of comorbidities, including cardiovascular disease, diabetes mellitus, hypertension, and certain non-AIDS-defining cancers, which occur more frequently and at an earlier age compared to HIV-negative individuals.

Frailty and multimorbidity are notably more common in aging PLWH, emphasizing the need for research into the mechanisms driving accelerated aging in this population. However, the exact pathways underlying these associations remain poorly understood, highlighting the necessity for further studies to clarify these mechanisms.

Despite the advancements on the identification of potentially strong biomarkers, no biomarker has yet been identified as specifically associated with multimorbidity in PLWH, representing a significant gap in understanding the biological mechanisms underlying these comorbidities. Biomarker research holds the potential to, for instance, stratify cardiovascular risk, optimize ART regimens to reduce inflammation and metabolic complications, and guide personalized interventions to improve health outcomes in this aging population.

The present invention is focused on solving this problem, and an innovative strategy for diagnosis, prognosis, monitoring and/or treatment of comorbidities in PLWH is herein provided.

### Description of the invention

### Brief description of the invention

The present invention refers to the diagnosis, prognosis, monitoring and/or treatment of comorbidities in PLWH.

The inventors of the present invention have identified the growth differentiation factor-15 (GDF-15) as a strong biomarker and therapeutic target of comorbidity in PLWH, particularly in the context of multimorbidity and aging. Such as it is shown in the Examples below, elevated GDF-15 levels were significantly associated with the presence of multimorbidity and correlated positively with age, years since diagnosis and duration of ART. The negative association with nadir CD4+ count suggests a link between GDF-15 levels and immune recovery, while lifestyle factors such as smoking, alcohol use and previous heroin use further influence GDF-15 levels expression.

Unlike GDF-15, the levels of sICAM-1, sVCAM-1 and sP-selectin showed no significant associations with multimorbidity or other clinical variables, except for a negative correlation between sP-selectin levels and age. This underscores the distinct role of GDF-15 in reflecting the interplay between chronic inflammation, aging and comorbidity in PLWH. The independent association of GDF-15 with multimorbidity, supported by multivariate analysis, positions this biomarker as a valuable tool for stratifying comorbidity risk in aging PLWH. Its utility in identifying individuals at higher risk of metabolic and cardiovascular complications could inform targeted interventions, optimized ART regimens and personalized care strategies to improve long-term outcomes in this vulnerable population.

On the other hand, these results open the possibility of using therapies directed against GDF-15, such as immunotherapy, to treat or prevent comorbidities in PLWH.

Therefore, the first embodiment of the present invention refers to an *in vitro* method for the prognosis and/or for monitoring the progression of PLWH, which comprises assessing the expression level or the amount of GDF-15 in a biological sample obtained from the patient, wherein the identification of a higher level of expression or amount of GDF-15, as compared with a pre-established threshold value measured in control subject who are not suffering from HIV infection, is an indication that the patients have poor prognosis or an increased burden of comorbidities.

The second embodiment of the present invention refers to an *in vitro* method for diagnosing or for identifying whether PLWH have an increased burden of comorbidities, which comprises assessing the expression level or the amount of GDF-15 in a biological sample obtained from the patient, wherein the identification of a higher level of expression or amount of GDF-15, as compared with a pre-established threshold value measured in control subjects who are not suffering from HIV infection, is an indication that the patients have an increased burden of comorbidities.

The third embodiment of the present invention refers to the *in vitro* use of GDF-15, or of a kit suitable for assessing the expression level or the amount of GDF-15, for the prognosis and/or for monitoring the progression of PLWH; or for diagnosing or for identifying whether PLWH have an increased burden of comorbidities.

In a preferred embodiment, comorbidities are selected from the group: cardiovascular disease, diabetes mellitus, hypertension, other viral co-infections (i.e. HCV, HBV), and/or non-AIDS-defining cancers.

In a preferred embodiment, the non-AIDS-defining cancers comprise: cancers of the lung, liver, kidney, anus, head and neck, and skin, as well as Hodgkin's lymphoma.

In a preferred embodiment, the biological sample is selected from: blood, serum or plasma.

On the other hand, the present invention is clear since a person skilled in the art would understand how to measure the level or amount of GDF-15 using well-established techniques. Furthermore, the skilled person would recognize that the detection of an elevated level of GDF-15 is carried out by comparing it to the level measured in control subjects, represented by individuals not suffering from the infection. Given the availability of standard methodologies for biomarker quantification and control group selection, the claimed invention is sufficiently clear and unambiguous.

Moreover, kindly note that the expression "increased burden of comorbidities" is clear and well established in the literature. In fact, it is common general knowledge that PLWH may experience an increased burden of comorbidities. This phenomenon has been widely documented in scientific literature and clinical practice, as HIV infection is associated with a higher prevalence of various comorbid conditions compared to the general population. These comorbidities include, but are not limited to, cardiovascular diseases, metabolic disorders such as diabetes and dyslipidemia, chronic kidney disease, osteoporosis, neurocognitive disorders, and an increased risk of certain malignancies. The higher burden of these conditions is attributed to a combination of chronic immune activation, inflammation, long-term effects of antiretroviral therapy, and other risk factors commonly found in this patient population.

The fourth embodiment of the present invention refers to an anti-GDF-15 antibody, or GDF-15 neutralizing antibody, for use in a method for the treatment and/or prevention of comorbidities in PLWH. In other word, the present invention also refers to a method for preventing and/or treating comorbidities in PLWH that comprises administering to the patient a pharmaceutically effective dose or amount of an anti-GDF-15 antibody, or GDF-15 neutralizing antibody, or a pharmaceutical composition comprising thereof.

In a preferred embodiment, the method for the treatment and/or prevention of comorbidities in PLWH are directed to those patients who have been identified and having poor prognosis or an increased burden of comorbidities according to the method explained in the first, second and third embodiment of the present invention.

In a preferred embodiment, the method for the treatment and/or prevention of comorbidities in PLWH comprises: i) Evaluating the patient by following the method explained in the first, second and third embodiment of the present invention and ii) Administering the anti GDF-15 antibody, or a pharmaceutical comprising comprise thereof, to the HIV-infected patient, once the patient has been identified and having a poor prognosis or an increased burden of comorbidities.

In a preferred embodiment, the anti-GDF-15 antibody is selected from: Visugromab, Ponsegromab, Cabiralizumab or AV-380.

So, the present invention also encompasses the use of immunotherapy targeting GDF-15 to prevent or treat comorbidities in PLWH. In particular, the invention includes approaches such as monoclonal antibodies, immune checkpoint inhibitors, therapeutic vaccines, or other immunomodulatory agents designed to enhance or modulate the immune response against the target. These strategies aim to either reduce disease progression or prevent its onset by leveraging the body's immune system to counteract the pathological effects associated with the target.

For the purpose of the present invention the following terms are defined:
- The term "comprising" means including, but not limited to, the elements listed. The use of "comprising" indicates that the specified elements are mandatory, but additional elements may also be present.
- The term "consisting of" means including only the elements explicitly listed, with no additional elements permitted. The phrase "consisting of" signifies that the specified elements are both necessary and exclusive.
- The term "diagnosis" refers to the process of identifying the presence of one or more comorbid conditions in an individual living with HIV. This involves evaluating clinical symptoms, medical history, and laboratory or imaging tests to detect diseases commonly associated with HIV, such as cardiovascular disease, metabolic disorders, chronic kidney disease, osteoporosis, neurocognitive impairments, and certain malignancies. The diagnosis may also include assessing biomarkers (such as it is indicated in the present invention), immune status, and the impact of long-term antiretroviral therapy to determine the patient's overall health status and disease progression.
- The term "prognosis" refers to the predicted course and outcome of one or more comorbid conditions in an individual living with HIV. This includes assessing the likelihood of disease progression, response to treatment, and overall impact on the patient's health and life expectancy. Prognosis is influenced by factors such as immune status (e.g., CD4+ T-cell count), viral load, duration of HIV infection, effectiveness of antiretroviral therapy, presence of chronic inflammation, and individual risk factors like age, lifestyle, and genetic predisposition. Understanding prognosis helps guide treatment decisions and long-term disease management strategies.
- The term "comorbidity" in the context of HIV patients refers to the presence of one or more additional diseases or medical conditions that coexist with HIV infection. These conditions, which may include cardiovascular disease, diabetes, chronic kidney disease, osteoporosis, neurocognitive disorders, other viral co-infections, and certain cancers, often arise due to chronic immune activation, inflammation, aging, or long-term effects of antiretroviral therapy. The term "multimorbidity" refers to the coexistence of multiple chronic conditions in an individual with HIV, beyond the primary infection. This is particularly relevant in aging HIV populations, where the combined impact of HIV-related and non-HIV-related conditions leads to complex healthcare needs and increased morbidity.
- The expression "pre-established threshold value measured in control subjects who are not suffering from HIV infection" refers to a specific reference value or cutoff point derived from measurements taken in individuals who are not infected with HIV. This value serves as a baseline for comparison to assess the presence or level of a biomarker or condition in PLWH. The pre-established threshold is typically determined using standard diagnostic methods or statistical techniques, such as mean, median, or other population-based metrics, obtained from a control group of healthy individuals. A person skilled in the art would be familiar with established methodologies for calculating such a threshold value, including the selection of appropriate control groups, statistical analysis, and interpretation of the results to ensure the threshold accurately reflects the normal range for the biomarker or condition in question.
- The expression "pharmaceutically effective dose or amount" refers to the quantity of a pharmaceutical agent, such as a drug or therapeutic compound, that is required to produce the desired therapeutic effect in a patient, with minimal side effects or toxicity. The effective dose is typically determined through clinical trials and is dependent on factors such as the drug's potency, the route of administration, the patient's condition, and individual factors like age, weight, and metabolism. The "pharmaceutically effective dose" is often described in terms of its ability to achieve a therapeutic outcome, such as symptom relief, disease prevention, or treatment efficacy, while maintaining safety and tolerability. A person skilled in the art would be familiar with methods to determine this dose based on pharmacokinetic and pharmacodynamic data, as well as clinical experience and regulatory guidelines. "Pharmaceutically effective dose or amount" in the context of immunotherapy for HIV patients refers to the specific quantity of an immunotherapeutic agent-such as a monoclonal antibody, immune checkpoint inhibitor, or therapeutic vaccine-that is required to achieve a therapeutic effect, which include reducing HIV-associated comorbidities or improving immune function. The effective dose is determined through clinical trials and considers factors like the drug's ability to modulate the immune response, reduce inflammation, or prevent or treat comorbid conditions commonly seen in HIV-infected individuals, such as cardiovascular disease, diabetes, and neurocognitive disorders. A person skilled in the art would understand how to calculate this effective dose based on clinical data, pharmacokinetic and pharmacodynamic principles, and the specific needs of HIV patients who often have multiple chronic conditions requiring careful management. The goal is to administer a dose that achieves the desired therapeutic response, balancing efficacy with safety to minimize adverse effects in patients with complex health profiles.

### Description of the figures

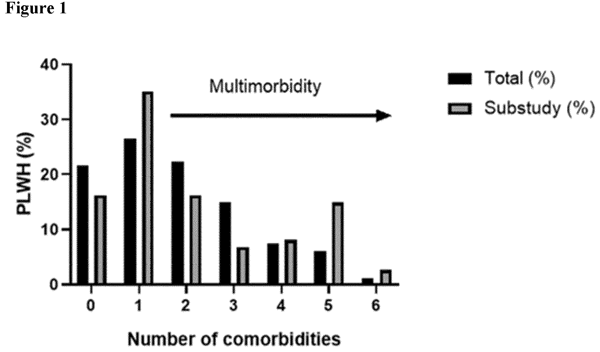

Figure 1. Distribution of comorbidities in the cohort sample and substudy population.

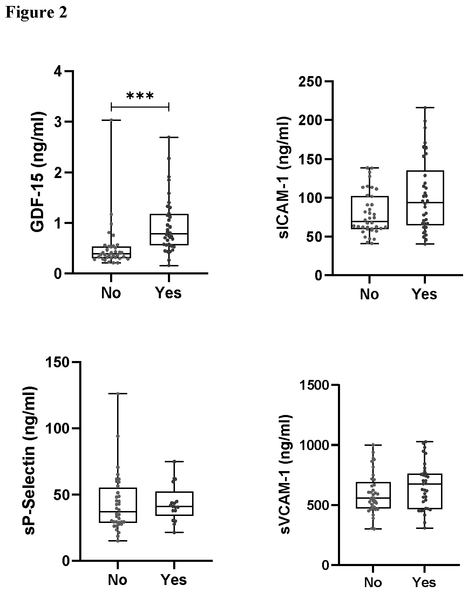

Figure 2. Correlation between biomarker levels and the presence of multimorbidity (2 or more comorbidities) in PLWH. Among the biomarkers analyzed, circulating GDF-15 protein levels were significantly elevated in PLWH with multimorbidity (M = 0.77 [0.54 - 1.19], n = 35), compared to PLWH without multimorbidity (M = 0.39 [0.31 - 0.54], n = 36; p<0.0001), suggesting its potential as a marker for identifying or monitoring multimorbidity in PLWH. While sICAM-1, sP-Selectin, and sVCAM-1 showed trends towards higher concentrations in the multimorbidity group, these differences did not reach statistical significance and warrant further investigation.

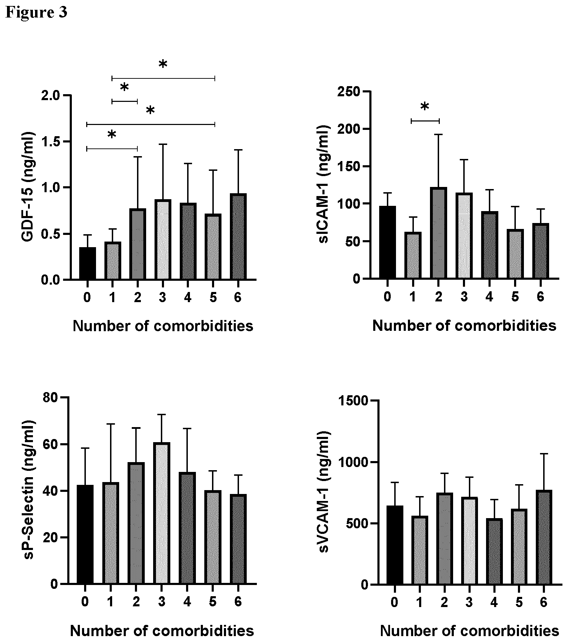

Figure 3. Levels of cardiovascular biomarkers by number of comorbidities.

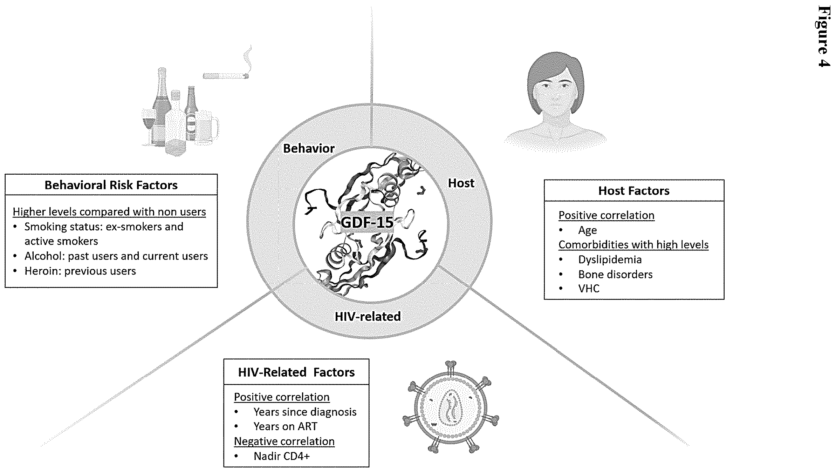

Figure 4. Factors associated with elevated GDF-15 levels. Elevated GDF-15 levels were positively correlated with age (ρ = 0.47; p < 0.001; n = 71), years since diagnosis (ρ = 0.337; p = 0.010; n = 57), and years on ART (ρ = 0.37; p = 0.005; n = 55). Conversely, GDF-15 levels were negatively correlated with nadir CD4+ count (ρ = -0.34; p = 0.004; n = 69). Lower GDF-15 levels were observed in PLWH who never smoked (M = 0.41; n = 23) compared with ex-smokers (M = 0.70; n = 14; p = 0.005) and active smokers (M = 0.63; n = 34; p = 0.003). Similarly, non-risk alcohol users (M = 0.45; n = 37) had lower levels than past users (M = 0.87; n = 11; p = 0.005) and current users (M = 0.55; n = 22; p = 0.015). GDF-15 levels were higher in previous heroin users (M = 0.93; n = 21) compared to non-users (M = 0.43; n = 49; p < 0.001). Elevated GDF-15 levels were also associated with dyslipidemia, bone disorders, and a history of hepatitis C virus infection.

### Detailed description of the invention

The present invention is illustrated by the following Examples, which are provided for illustrative purposes only and are not intended to limit the scope of protection.

### Example 1. Material and methods

### Example 1.1. Study design

This study was designed as a cross-sectional investigation, focusing on the epidemiological and clinical characterization of PLWH. Additionally, molecular characterization of inflammatory and endothelial function biomarkers was conducted. The study population comprised PLWH under follow-up at the Álvaro Cunqueiro Hospital in Vigo, who are part of the Infectious Diseases Cohort registered in the IISGS Biobank (Biobank reference B.0000802).

### Example 1.2. Study population

Participants were recruited during internal medicine consultations in the HIV units of the Álvaro Cunqueiro Hospital in Vigo. Eligible participants were included in the Infectious Diseases Cohort of the IISGS Biobank if they met specific inclusion and exclusion criteria. The study population was stratified into two groups: individuals with multimorbidity (defined as having two or more comorbidities) and those without multimorbidity (defined as having one or no comorbidities). Eligible participants were adults diagnosed with HIV who had been on stable antiretroviral therapy (ART) for at least one year and maintained an undetectable viral load (plasma viral load of <50 HIV RNA copies/mL) for at least one year. All participants were required to have attended at least one consultation at the HIV unit between 2020 and 2023. Patients who had not attended any consultation during this period were excluded.

### Example 1.3. Biomarker quantification

To assess markers of endothelial function and inflammation, concentrations of sVCAM-1, sICAM-1, sP-selectin and GDF-15 (measured in ng/mL) were quantified using a multiplex bead-based immunoassay (MILLIPLEX^{®} Cardiovascular Disease Panel 2; Merck EMD Millipore, Billerica, MA, USA). Data points with a coefficient of variation exceeding 20% were excluded from the analysis to ensure the accuracy and reliability of the results.

### Example 1.4. Data Analysis

For the descriptive study, values were expressed as absolute numbers and percentages and as medians and interquartile ranges (IQRs). A bivariate analysis and multiple binary logistic regression were conducted. IBM SPSS Statistics for Windows version was used for all calculations. All statistical tests were 2-sided and P value of < 0.05 was considered statistically significant

### Example 1.5. Ethical Considerations

The study adhered to ethical standards and all participants in the Infectious Diseases Cohort provided informed consent for the use of their data in research. Informed consent was obtained during routine clinical follow-up. The project received ethical approval from the Galician Committee on the Ethics of Research Involving Medicinal Products (CEIm-G) under approval notice 2024/315.

### Example 2. Results

### Example 2.1. Clinical and epidemiological characteristics of the cohort

A total of 268 PLWH met the inclusion criteria and were included in the study, of whom 180 (70.1 %) were men, 77 (28.7 %) women and 3 (1.1 %) transexual. The average age was 49.8 years, ranging from 19 to 88 years, with 50.7% of participants being over 50 years old. The majority were of Spanish origin (84 %), followed by Latin American (9.7 %) **(Table 1).**

**Table 1. Socio-demographics characteristics of the study population**

| | **Total** (N = 268) n (%) | **Substudy** (N=74) n (%) |
|---|---|---|
| **Age** (years) | 49.8 (19-88) | 53 (28-81) |
| 18-30 | 17 (6.3) | 5 (6.8) |
| 31-49 | 115 (42.9) | 20 (27.0) |
| ≥ 50 | 136 (50.7) | 49 (66.2) |

| **Sex** | | |
|---|---|---|
| Men | 180 (70.1) | 55 (74.3) |
| Women | 77 (28.7) | 19 (25.7) |
| Transexual | 3 (1.1) | 0 (0.0) |

| **Origin** | | |
|---|---|---|
| Spanish | 225 (84.0) | 62 (83.8) |
| Latin America | 26 (9.7) | 8 (10.8) |
| African | 7 (2.6) | 2 (2.7) |
| Other | 9 (3.4) | 2 (2.7) |
| Unknown | 1 (0.4) | 0 (0.0) |

The average time living with HIV among participants was 17.8 (±9.84) years, with a median time from diagnosis to ART initiation of 49 days (range: 8-696 days). The predominant modes of disease transmission categories were men who has sex with men (35.8 %). Almost all (98.1 %) were on ART with undetectable viral load (91.7 %). The median time on ART was 15 (±8.96) years. The average latest CD4+ was 746 cells/µl. The most common ART regimen was based on integrase inhibitors (INIs), prescribed to 224 participants (83.6 %). The most frequently used combinations were bictegravir, emtricitabine and tenofovir alafenamide (BIC/FTC/TAF) (97, 36.2 %), dolutegravir plus lamivudine (DTG/3TC) (85, 31.7 %) and dolutegravir with rilpivirine (DTG/RPV) (30, 11.2 %). This was followed by protease inhibitors regimen (PIs) (16, 6.0 %), a combination of INIs and PIs (13, 4.9 %) and regimen based on nucleoside reverse-transcriptase inhibitors (NRTIs) combined with non-nucleoside reverse-transcriptase inhibitors (NNRTIs) (13, 4.9 %) **(Table 2).**

**Table 2. Clinical characteristics of the study population**

| | | **Total** (N = 268) n (%) | **Substudy** (N = 74) n (%) |
|---|---|---|---|
| **Time living with HIV** (years) | | | |
| | < 10 | 56 (20.9) | 18 (30.5) |
| | 10-19 | 71 (26.5) | 16 (27.1) |
| | > 20 | 94 (35.1) | 25 (42.4) |
| **Time on ART** (years) | | 15.2 (±8.96) | 15.2 (±9.84) |

| **ART** | | | |
|---|---|---|---|
| INI based regimen | | 224 (83.6) | 70 (94.6) |
| | BIC/FTC/TAF^{a} | 97 (36.2) | 12 (16.2) |
| | DTG/3TC^{b} | 85 (31.7) | 37 (50.0) |
| | DTG/RPV^{c} | 30 (11.2) | 16 (21.6) |
| | Other INI based regimen | 12 (4.5) | 5 (6.8) |
| IP based regimen | | 13 (4.9) | 1 (1.4) |
| NRTI^{d} + NNRTI^{e} regimen | | 13 (4.9) | 3 (4.1) |

| **HIV transmission category** | | | |
|---|---|---|---|
| | Men who have sex with men | 96 (35.8) | 28 (37.8) |
| | Heterosexual | 89 (33.2) | 23 (31.1) |
| | Intravenous drug user | 69 (25.7) | 22 (29.7) |
| | Blood product transfusion | 5 (1.9) | 0 (0.0) |
| | Vertical transmission | 6 (2.2) | 0 (0.0) |
| | Unknown | 3 (1.1) | 1 (1.4) |
| **CD4+ lymphocyte nadir** (cells/µl) | | 254 (110 - 406) | 223 (87 - 378) |
| **CD4+ lymphocyte at diagnosis** (cells/µl) | | 445 (205 - 655) | 336 (205 - 649) |
| **HIV viral load at diagnosis** (copies/ml) | | 57554 (16300 - 2950000) | 65950 (13687 - 366500) |

| | | | |
|---|---|---|---|
| ^{a}BIC/FTC/TAF: bictegravir/emtricitabine/tenofovir alafenamide; ^{b}DTG/3TC: dolutegravir/lamivudine; ^{c}DTG/RPV: dolutegravir/rilpivirine; ^{d}NRTI: nucleoside reverse transcriptase inhibitor; ^{e}NNRTI: non-nucleoside reverse transcriptase inhibitor. | | | |

More than half of the participants (139, 51.9%) presented with multimorbidity, defined as the presence of two or more chronic health conditions excluding HIV **(****Figure 1****).** The most frequent comorbidities included bone disorders (osteopenia and osteoporosis) (41.4%), past hepatitis B virus (HBV) infection (34.0%), past hepatitis C virus (HCV) infection (31.3%), arterial hypertension (15.3%) and chronic obstructive pulmonary disease (COPD) (9.3%) **(Table 3).** Dyslipidemia was highly prevalent in the cohort, affecting 26.1% of participants. The presence of dyslipidemia was associated with CD4 lymphocyte count at diagnosis. We identified significantly lower CD4 lymphocyte count at HIV diagnosis in PLWH with dyslipidemia compared to those without dyslipidemia (99.7 vs. 124.7 cells/µL; SD = 461.6; p = 0.013).

All comorbidities were statistically more frequent among individuals over 50 years of age, except for COPD, which had similar rates between younger and older adults (9.8 % vs. 8.8 %; p = 0.773). All clinical characteristics were similar between men and women, except for a significantly higher prevalence of HBV past infection among men (40.4 % vs 22.1 %; p = 0.004).

Regarding mental health, 41 participants (15.3 %) were receiving clinical follow-up for substance use disorders, primarily associated with opioids (33, 12.3 %), cocaine (9, 3.4 %) or alcohol (8, 3.0 %). The most common psychiatric comorbidities were anxiety disorders, affecting 30 PLWH (11.2 %), including mixed anxiety-depressive disorder (15, 5.6 %), generalized anxiety disorder (11, 4.1 %) and severe stress reactions and adjustment disorders (9, 3.4 %) **(Table 3).**

**Table 3. Comorbidity-related characteristics of the study population**

| | | **Total** (N = 268) n (%) | **Substudy** (N = 74) n (%) |
|---|---|---|---|
| **Most common comorbidities** | | | |
| Arterial hypertension | | 41 (15.3) | 17 (23.0) |
| Diabetes mellitus | | 17 (6.4) | 5 (6.8) |
| Dyslipidemia | | 70 (26.1) | 21 (28.4) |
| Bone disorders | | | |
| | Osteopenia | 79 (41.6) * | 27 (47.4) * |
| | Osteoporosis | 31 (16.3) * | 11 (19.3) * |
| Hepatitis B virus infection | | | |
| | Chronic active HBV infection | 3 (1.1) | 1 (1.4) |
| | Past HBV infection | 91 (34.0) | 26 (35.1) |
| Hepatitis C virus infection | | | |
| | Chronic active HCV infection | 3 (1.1) | 1 (1.4) |
| | Past HCV infection | 84 (31.3) | 22 (29.7) |
| Ischemic heart disease | | 9 (3.4) | 2 (2.7) |
| Congestive heart failure | | 2 (0.7) | 1 (1.4) |
| Peripheral arterial disease | | 7 (2.6) | 3 (4.1) |
| Cerebrovascular disease | | 8 (3.0) | 5 (6.8) |
| Chronic obstructive pulmonary disease (COPD) | | 25 (9.3) | 5 (6.8) |
| Active solid organ cancer | | 12 (4.5) | 6 (8.1) |

| **Mental health comorbidities** | | | |
|---|---|---|---|
| Substance use disorders | | 41 (15.3) | 12 (16.2) |
| | Opioids | 33 (12.3) | 11 (14.9) |
| | Cocaine | 9 (3.4) | 1 (1.4) |
| | Alcohol | 8 (3.0) | 0 (0.0) |
| | Hypnotic and sedatives | 8 (1. 1) | 0 (0.0) |
| | Cannabinoids | 5 (1.9) | 1 (1.4) |
| | Other psychoactive substances | 2 (0.7) | 1 (1.4) |
| | Harmful use of multiple drugs (no dependence detected) | 2 (0.7) | 1 (1.4) |
| Schizophrenia | | 5 (1.9) | 0 (0.0) |
| Mood disorders (affective) | | 10 (3.7) | 1 (1.4) |
| | Bipolar disorder | 2 (0.7) | 0 (0.0) |
| | Recurrent major depressive disorder | 2 (0.7) | 1 (1.4) |
| | Symptoms of depressive disorder | 6 (2.3) | 0 (0.0) |
| Anxiety disorders | | 30 (11.2) | 10 (13.5) |
| | Mixed anxiety-depressive disorder | 15 (5.6) | 1 (5.4) |
| | Generalized anxiety disorder | 11 (4.1) | 4 (5.4) |
| | Severe stress reactions and adjustment disorders | 9 (3.4) | 1 (1.4) |
| | Panic disorders | 2 (0.7) | 2 (2.7) |
| | Other anxiety disorders | 7 (2.6) | 2 (2.7) |
| Sleep disorders | | 11 (4.1) | 2 (2.7) |
| Alzheimer's dementia | | 1 (0.4) | 1 (1.4) |
| Personality disorders | | 3 (1.1) | 1 (1.4) |
| Paraphilias | | 1 (0.4) | 1 (1.4) |
| Attention deficit hyperactivity disorder | | 1 (0.4) | 1 (1.4) |

Substance use disorders associated with opioids were the only mental health condition significantly more prevalent among adults over 50 years compared to younger participants (69.7 % vs. 30.3 %, p = 0.025). Severe stress reactions and adjustment disorders were more common in women than in men (62.5% vs. 37.5%; p = 0.048). Conversely, substance use disorders associated with opioid use were significantly higher in men compared to women (87.9% vs. 12.2%; p = 0.024), while those associated with psychoactive substance use also tended to be higher in men (84.4% vs. 14.6%; p = 0.26).

We also assessed the lipid profile of the cohort **(Table 4).**

**Table 4. Lipid profile of the study population**

| | **Total** (N = 268) Mean (±SD^{a}) / median (IQR^{b}) | **Substudy** (N = 74) Mean (±SD) / median (IQR) |
|---|---|---|
| **Total cholesterol** (mg/dl) | 185.97 (± 37.90) | 182.4 (± 37.90) |
| **HDL cholesterol** (mg/dl) | 48.00 (39.0 - 58.3) | 48.00 (39.0 - 59.0) |
| **LDL cholesterol** (mg/dl) | 108.55 (± 28.72) | 109.0 (± 27.7) |
| **Triglyceride** (mg/dl) | 115 (82.0 - 155.8) | 111.5 (76.0 - 156.0) |

| | | |
|---|---|---|
| ^{a}SD: standard deviation; ^{b}IQR: interquartile range. | | |

A weak negative correlation was observed between the nadir CD4+ count and HDL cholesterol levels (ρ = -0.192; p = 0.004; n = 221). No significant correlations were found between other lipid profile variables and CD4 count at diagnosis, or HIV RNA levels at diagnosis. It should be noted that 67 individuals (25.0%) were receiving lipid-modifying treatments, including 54 (20.1%) on HMG-CoA reductase inhibitors as monotherapy, 5 (1.9%) on fibrates, 4 on ezetimibe and 5 on combinations of lipid-modifying agents. Additionally, 4 individuals (1.4%) were on combinations of lipid-modifying agents with other drugs.

### Example 2.2. Cardiovascular biomarkers in aging people living with HIV

### Example 2.2.1. Clinical and epidemiological characteristics of the study subpopulation

We analyzed plasma levels of GDF-15, sVCAM-1, sICAM-1 and sP-selectin in 74 PLWH. The biomarker values for each group are presented in **Table 5.**

**Table 5. Concentration of biomarkers in PLWH**

| | **Without multimorbidity** | | **Multimorbidity** | | p |
|---|---|---|---|---|---|
| | n | median [IQR^{a}] ng/ml | n | median [IQR] ng/ml | |
| **GDF-15** | 36 | 0.39 [0.31 - 0.54] | 35 | 0.77 [0.54 - 1.19] | **<0.001** |
| **sICAM-1** | 38 | 69.62 [60.62 - 102.13] | 34 | 94.11 [65.25 - 135.10] | 0.054 |
| **sP-Selectin** | 37 | 38.56 [29.20 - 54.02] | 32 | 47.08 [38.56 - 59.62] | 0.060 |
| **sVCAM-1** | 37 | 559.43 [477.72 - 692.97] | 35 | 677.99 [472.64 - 763.34] | 0.124 |

| | | | | | |
|---|---|---|---|---|---|
| ^{a}IQR: interquartile range. | | | | | |

The demographic characteristics of the studied subpopulation are consistent with those of the overall cohort **(Table 1).** Additionally, the clinical profiles of the substudy population and the overall cohort are comparable, as shown in **Tables 2, 3** and **4.** The distribution of the number of comorbidities in both study groups is represented in **Figure 1****.**

### Example 2.2.2. GDF15 levels correlation with multimorbidity and aging with HIV

As shown in **Figure 2****,** circulating GDF-15 protein levels were significantly elevated in PLWH, with the highest levels observed in those with multimorbidity (M = 0.77 [0.54 - 1.19], n = 35), compared to PLWH without multimorbidity (M = 0.39 [0.31 - 0.542], n = 36; p<0.001). Notably, GDF-15 was the only biomarker significantly elevated in PLWH with multimorbidity. **Figure 3** illustrates the correlation between biomarkers levels and the number of comorbidities in PLWH, highlighting GDF-15 as the only robust biomarker of multimorbidity.

Notably, among PLWH, elevated GDF-15 levels showed a positive correlation with age (ρ = 0.47; p < 0.001; n = 71), years since diagnostic (ρ = 0.337; p = 0.010; n = 57) and years on ART (ρ = 0.37; p = 0.005; n = 55). Conversely, GDF-15 levels were negatively correlated with nadir CD4+ count (ρ = -0.34; p = 0.004; n = 69). Also, we found lower levels of GDF-15 in PLWH who never smoke (M = 0.41, n = 23) compared with ex-smokers (M = 0.70; n = 14, p = 0.005) and active smokers (M = 0.63, n = 34; p =0.003), in non-risk alcohol users (M = 0.45, n = 37) compared with past users (M = 0.87, n = 11; p = 0.005) and current users (M = 0.55, n = 22; p = 0.015) and higher levels of GDF-15 in previous heroin users (M = 0.93, n = 21) compared to non-users (M = 0.43, n = 49; p < 0.001) and PLWH with (M = 0.75, n = 21) compared to without dyslipidemia (M = 0.47, n = 50, p = 0.015) **(****Figure 4****).**

We did not observe correlation between increases in GDF-15 levels and baseline viral load prior to ART, levels of triglycerides, total cholesterol, HDL cholesterol or LDL cholesterol in PLWH. Moreover, there were no differences in the GDF-15 levels between men (n = 53) and women (n = 18) with HIV; patients with (n = 16) and without (n = 55) hypertension; and patients with (n = 5) and without diabetes (n = 66), or PLWH who received different ART regimens.

Multivariate analysis identified multimorbidity as the only independent factor associated with GDF-15 [β=.38, (95 % CI: 0.02-0.59; p = 0.039; R² = 35.0 %].

### Example 2.2.3. sICAM-1, sVCAM-1 and sP-selectin

We did not observe significant differences in the levels of sICAM-1, sVCAM-1 and sP-selectin between PLWH with and without multimorbidity **(Table 5).** Correlation analysis conducted for all PLWH (without stratification by multimorbidity) revealed a negative correlation between sP-selectin levels and age (ρ = -0.273; p = 0.023; n = 69). However, no correlations were observed between sICAM-1, sVCAM-1, or sP-selectin levels and years since diagnosis or duration on ART. Additionally, no significant correlations were found for sICAM-1 or sVCAM-1 levels with other variables.

## Claims

1. *In vitro* method for the prognosis and/or for monitoring the clinical progression of people living with human immunodeficiency virus (HIV) (PLWH), which comprises assessing the expression level or the amount of growth differentiation factor-15 (GDF-15) in a biological sample obtained from the patient, wherein the identification of a higher level of expression or amount of GDF-15, as compared with a pre-established threshold value measured in control subjects without HIV infection, is an indication that the patients have poor prognosis or an increased burden of comorbidities.

2. *In vitro* method for diagnosing or for identifying whether PLWH have an increased burden of comorbidities, which comprises assessing the expression level or the amount of growth differentiation factor-15 (GDF-15) in a biological sample obtained from the patient, wherein the identification of a higher level of expression or amount of GDF-15, as compared with a pre-established threshold value measured in control subjects who are not suffering from HIV infection, is an indication that the patients have an increased burden of comorbidities.

3. *In vitro* use of GDF-15, or of a kit suitable for assessing the expression level or the amount of GDF-15, for the prognosis and/or for monitoring the progression of PLWH; or for diagnosing or for identifying whether PLWH have an increased burden of comorbidities.

4. *In vitro* method, or *in vitro* use, according to any of the previous claims, wherein the comorbidities are selected from the group: cardiovascular disease (CVD), diabetes mellitus, hypertension and/or non-AIDS-defining cancers.

5. *In vitro* method, or *in vitro* use, according to any of the previous claims, wherein the non-AIDS-defining cancers comprise: cancers of the lung, liver, kidney, anus, head and neck, and skin, as well as Hodgkin's lymphoma.

6. *In vitro* method, or *in vitro* use, according to any of the previous claims, wherein the biological sample is selected from: blood, serum or plasma.

7. Anti GDF-15 antibody, or GDF-15 neutralizing antibody, for use in a method for the treatment and/or prevention of comorbidities in PLWH.

8. Anti GDF-15 antibody, or GDF-15 neutralizing antibody, for use, according to claim 7, in a method for the treatment and/or prevention of comorbidities in PLWH who have been identified and having poor prognosis or an increased burden of comorbidities according to the method of claims 1 to 6.

9. Anti GDF-15 antibody, or GDF-15 neutralizing antibody, for use, according to any of the claims 7 or 8, for use in a method for the treatment of comorbidities in PLWH, wherein the method comprises: i) Evaluating the patient by following the method of claims 1 to 6 and ii) Administering the anti GDF-15 antibody, or a pharmaceutical comprising comprise thereof, to the HIV-infected patient, once the patient has been identified and having a poor prognosis or an increased burden of comorbidities.

10. Anti GDF-15 antibody for use, according to any of the claims 7 to 9, selected from: Visugromab, Ponsegromab, Cabiralizumab or AV-380.
